# EUROPÄISCHE PATENTSCHRIFT

(11) **EP 1 667 640 B1**
(45) Veröffentlichungstag und Bekanntmachung des Hinweises auf die Patenterteilung: **05.08.2009**
(21) Anmeldenummer: 04765108.8
(22) Anmeldetag: 11.09.2004
(51) Int. Cl.: A61K 8/18, A61K 8/99, A61Q 5/12, A61Q 19/00

(54) **HAUT- UND HAARPFLEGEZUBEREITUNG ENTHALTEND PROTEINHYDROLYSATE**
SKIN AND HAIR CARE PREPARATION CONTAINING ROTEIN HYDROLYSATS
PREPARATION DE SOIN POUR LA PEAU ET LES CHEVEUX, CONTENANT DES HYDROLYSATS DE PROTEINES

(30) Priorität: 22.09.2003 DE 10344166; 20.11.2003 DE 10354316
(43) Veröffentlichungstag der Anmeldung: 14.06.2006
(73) Patentinhaber: JUVENA (International) AG, 8604 Volketswil / Zürich (CH)
(72) Erfinder: PICANO, Roger, CH-8472 Seerzach (CH); GOHLA, Sven, 21077 Hamburg (DE)
(74) Vertreter: Hartmann, Jost
(86) Internationale Anmeldenummer: PCT/EP2004/010185
(87) Internationale Veröffentlichungsnummer: WO 2005/032505

(56) Entgegenhaltungen:
- EP-A- 0 841 065
- WO-A-98/25584
- WO-A-20/04080426
- FR-A- 2 801 788
- PATENT ABSTRACTS OF JAPAN Bd. 1996, Nr. 04, 30. April 1996 (1996-04-30) & JP 07 330560 A (SHISEIDO CO LTD), 19. Dezember 1995 (1995-12-19)
- ANONYMOUS: "Seide aus dem Meer" INTERNET ARTICLE, [Online] Januar 2001 (2001-01), XP002314126 Gefunden im Internet: URL:http://www.impag.de/german/kosmetik/in dex.htm?http://www.impag.de/german/kosmeti k/z8seide.htm> [gefunden am 2004-01-18]
- PATENT ABSTRACTS OF JAPAN Bd. 2000, Nr. 10, 17. November 2000 (2000-11-17) & JP 2000 191446 A (LION CORP), 11. Juli 2000 (2000-07-11)
- DATABASE PROMT [Online] STN INTERNATIONAL; XP002314127 Database accession no. 2004:76834 & "The magic of molluscs" EUROPAEN COSMETIC MARKETS, Bd. 21, Nr. 2, Februar 2004 (2004-02), Seite 75,

## Beschreibung

Die Erfindung betrifft kosmetische Zubereitungen, insbesondere Haarpflegezubereitungen oder Haarpflegemittel, enthaltend Proteinhydrolysate aus Seide, Pashmina, Cashmere-Wolle, Merinowolle und/oder Mohair, Extrakt aus den Muschelfäden der Miesmuschel, den so genannten Byssus-Fäden, und Sericin und/oder Sericinhydrolysate. Die Zubereitungen sind mild zu Haut und Haaren und führen zu einer Verbesserung der Haarstruktur als auch der physikalisch-optischen Haareigenschaften.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt es eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft seinerseits ist aus drei Schichten aufgebaut: einem zentralen Teil - dem sogenannten Haarmark (Medulla), welches allerdings beim Menschen zurückgebildet ist und oft gänzlich fehlt - ferner dem Mark (Cortex) und der äußeren, bis zu zehn Lagen starken Schuppenschicht (Cuticula), die das ganze Haar umhüllt.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf. Insbesondere die Schuppenschicht als Außenhülle des Haares, aber auch der innere Bereich unterhalb der Cuticula sind besonderer Beanspruchung durch Umwelteinflüsse ausgesetzt.

Wesentliche Einflüsse für den Qualitätsverlust eines Haares während seiner Alterung sind der Einfluß des Sonnenlichts, mechanische Belastungen durch intensives Kämmen oder Bürsten, aber auch Haarbehandlungen, wie Haarfärbungen und insbesondere Blondierungen sowie Haarverformungen, beispielsweise Dauerwellverfahren. Besonders oxidative Belastungen führen demnach häufig zu einer Schädigung des Haares.

Sowohl UV-A- als auch UV-B-Strahlung haben einen schädigenden Einfluß auf das Haar, der sich beispielsweise darin äußert, dass bestimmte Aminosäuren wie Cystin und Methionin abgebaut oder Schwefel-Schwefel-Bindungen des Keratins gespalten werden, was im schlimmsten Fall eine Zerstörung des Haars zur Folge haben kann. Weiterhin stellen Haar und Kopfhaut Teile des Körpers dar, die aufgrund ihrer Position beim Aufenthalt im Freien einer erheblichen Menge an UV-Strahlung ausgesetzt sind.

Ein Ziel der Haarpflege und damit auch der vorliegenden Erfindung ist es, den Naturzustand des frisch nachgewachsenen Haares über einen möglichst langen Zeitraum zu erhalten und im Fall eines Verlusts wieder herzustellen. Seidiger Glanz, geringe Porosität und ein angenehmes, glattes Gefühl gelten als Merkmale für natürliches, gesundes Haar.

Seit Ende des vergangenen Jahrhunderts werden Produkte zur Haarpflege gezielt entwickelt. Dies führte zu einer Vielzahl von Präparaten sowohl für die allgemeine Haarpflege als auch zur Behebung von Anomalien des Haares und der Kopfhaut. Im allgemeinen werden heutzutage Haarpflegekosmetika verwendet, welche entweder dazu bestimmt sind, nach dem Einwirken aus dem Haar wieder ausgespült zu werden, oder welche auf dem Haar verbleiben sollen. Letztere können so formuliert werden, dass sie nicht nur der Pflege des einzelnen Haars dienen, sondern auch das Aussehen einer Frisur insgesamt verbessern. Solcherart gepflegtes Haar zeichnet sich durch einen angenehmen Griff, natürlichen Glanz, vermehrte Fülle, Geschmeidigkeit und somit gute Frisierbarkeit und Festigkeit und somit gutem Frisurensitz aus.

Produkte die ausschließlich der Pflege des Haares dienen, werden allgemein als Haarkonditioniermittel oder Conditioner bezeichnet. Diese können nach einer mehr oder weniger langen Verweilzeit auf dem Haar ausgespült werden (Rinse-off Produkte, z.B. Spülungen, Haar-Kuren) oder sie verbleiben nach der Anwendung auf dem Haar (Leaveon Produkte). Die Produkte können verschiedene Konsistenzen haben, so dass sie ganz unterschiedlich appliziert werden können. Es können Emulsionen oder Gele sein oder dünnflüssige Lösungen, die z.B. über Sprühapplikationen aufgebracht werden, oder Schäume, die z.B. durch geeignete Druckgaspackungen oder spezielle Schaumpumpen bei der Applikation erzeugt werden. Cremige, trübe und klar transparente Produkte sind im Markt zu finden.

Je nach Verwendungszweck findet man ganz unterschiedliche Wirkstoffe oder Kombinationen von Wirkstoffen in solchen Conditionern. Manche, die eher dem Schutz des Haares dienen, wie Antioxidantien oder UV-Filter, andere die das Haar geschmeidig machen wie z.B. kationische Tenside. Eine immer größere Bedeutung bekommen polymere Wirkstoffe, die je nach Art, Molgewicht und Ladung ganz unterschiedliche Eigenschaften haben. Im Vordergrund steht dabei jedoch eindeutig eine Verbesserung der Oberflächenbeschaffenheit des Haares.

Trotz einer großen Vielfalt von Produkten, die dem Verbraucher zur Verfügung stehen, sind einige Nachteile noch nicht restlos beseitigt. So zeigen Polymere, die dem Haar eher eine gewisse Festigkeit und somit Volumen geben häufig ein schlechtes Griffempfinden und eine schlechte Kämmbarkeit; Polymere, die das Haar geschmeidig machen führen häufig zu einer Beschwerung, was mit mangelndem Volumen verbunden ist. Auch der Einsatz von Stärkederivaten konnte in der Vergangenheit den geschilderten Nachteilen des Standes der Technik nicht abhelfen.

Gelförmige Zubereitungen des Standes der Technik haben noch eine Reihe von weiteren Nachteilen: Meist werden zur Verdickung der Zubereitungen Carbomere, d.h. Polyacrylate oder Cellulosederivate eingesetzt. Derartige Zubereitungen weisen jedoch in der Regel ein trübes Aussehen auf. Auch lassen sich in die Zubereitungen des Standes der Technik kaum salzartige Pflegestoffe und keine ionischen Filmbildner einarbeiten, da dies zu Unverträglichkeiten (Substanzausscheidungen, Ausfällungen, Viskositätsverlusten etc.) führt. Auch die sensorischen und taktilen Eigenschaften herkömmlicher mit Carbomeren oder Cellulosederivaten verdickten Zubereitungen entsprechen nicht den Anforderungen der Verbraucher, da sie als schmierig und glitschig empfunden werden.

Ziel der vorliegenden Erfindung ist es auch Formulierungsvarianten zu schaffen, die verbesserte sensorische und taktile Eigenschaften aufweisen als Zubereitungen des Standes der Technik. Insbesondere gilt es auch Zubereitungen zur Verfügung zu stellen, deren Biopolymerbestandteile in deutlichem Maße aus den Formulierungen zur Haar - und Kopfbodenpflege freigesetzt werden können.

Bei der Körperpflege des Menschen spielt auch die Haarwäsche eine zentrale Rolle. Die Reinigung der Haare und der Kopfhaut von körpereigenem Fett, Hautabschilferungen, Schmutz und Gerüchen entspricht einem Grundbedürfnis des Menschen.

Zur Befriedigung dieses Bedürfnisses stand dem Menschen bis ins 20.Jahrhundert hinein allein Seife zur Verfügung, die aufgrund ihres alkalischen pH-Wertes für die Kopfhaut und die Augenschleimhäute wenig verträglich war und häufig Ablagerungen von Kalkseifen im Haar zurück ließ. In den dreißiger Jahren des 20.Jahrhunderts erblickte das erste alkylsulfathaltige Shampoo das Licht der Welt. Seit Mitte der sechziger Jahre erobern Alkylethersulfate und andere Tenside den Shampoomarkt. Mit ihnen können die Nachteile von seifenhaltigen Zubereitungen vermieden werden. Heutzutage müssen moderne Shampoos das Haar nicht nur ,reinigen und gut verträglich sein. Vielmehr sollen sie das Haar auch pflegen und seine Frisierbarkeit und optische Attraktivität erhöhen.

Haarshampoos enthalten eine Vielzahl unterschiedlicher Komponenten um den einzelnen Anforderungen an das Produkt gerecht zu werden:

Die Reinigungskraft der Shampoos wird bewirkt durch die Anwesenheit von anionischen, amphoteren und nichtionischen Tensiden als oberflächenaktive Verbindungen in den Zubereitungen. Tenside sorgen darüber hinaus für das Schaumvermögen der Haarreinigungsmittel. Wichtig bei der Auswahl der Tenside ist darüber hinaus ihre Unempfindlichkeit gegenüber der Wasserhärte, ihre biologische Abbaubarkeit, ihre Verträglichkeit mit anderen Komponenten der Zubereitung sowie ihr Preis. Ein viel verwendetes Shampootensid ist beispielsweise Alkylethersulfat.

Darüber hinaus enthalten Shampoos eine Reihe von Konsistenzregulatoren, die der. Zubereitung die gewünschte Viskosität verleihen. Diese Verdicker bewirken eine Vergrößerung der Tensidmicellen bzw. eine Quellung der Wasserphase der Zubereitung. Verdicker können aus chemisch sehr unterschiedlichen Stoffklassen gewählt werden. So werden u.a. Elektrolyte (z.B. Natriumchlorid), Alkanolamide (z.B. Fettsäure-Monoethanolamide), niedrig ethoxylierte Fettalkohole (z.B. Diethylenglycolmonolaurylether), hochethoxylierte Ether, Ester und Diester sowie polymere Verdicker eingesetzt. Zu den polymeren Verdickern zählen beispielsweise Celluloseether. Darüber hinaus finden auch Polyacrylate und Hydrokolloide als Verdicker Verwendung. Polymere Verdicker haben den großen Vorteil, dass die durch sie erzeugte Viskosität weitgehend temperaturunabhängig ist.

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. Hierzu zählen neben UV-Absorbern, Vitaminen oder Pflanzenextrakten auch sogenannte Haarkonditionierer (engl. conditioner), welche das Haar pflegen und seine Kämmbarkeit und seinen Griff verbessern sowie seinen Glanz erhöhen. Konditionierer ziehen, im Gegensatz zu den meisten anderen Bestandteilen von Shampoos, auf das Haar auf und verbleiben dort nach dem Spülen. Sie lagern sich aufgrund ihres Molekülaufbaus an die Schadstellen der Cuticula des Haares und glätten das Haar. Dadurch wird das Haar weniger rauh und spröde, die Frisur bekommt deutlich mehr Glanz und läßt sich leichter kämmen. Auch wird das Haar weniger empfindlich für eine elektrostatische Aufladung. Die wichtigsten haarkonditionierenden Substanzen, in der Regel kationische Polymere, stellen die polymeren quatären Ammoniumverbindungen dar. Auch können kationische Cellulosederivate und' Polysaccharide eingesetzt werden. Weiterhin werden auch Silikonverbindungen zur Konditionierung eingesetzt,

Aber auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Der Stande der Technik lässt es an Shampooformulierungen mangeln, welche dem geschädigten Haar in befriedigender Weise Pflege zukommen ließen. Aufgabe ist es daher, auch diesen Nachteilen des Standes der Technik Abhilfe zu schaffen.

Die Aufgabe der vorliegenden Erfindung ist daher auch eine alternative kosmetische Reinigungs- und Pflegezubereitung bereit zu stellen, die sich insbesondere durch gute Haut- und Haarverträglichkeit, gutes Schaumverhalten und angenehme Haarpflege und - frisieren auszeichnen.

Gelöst wird dieses Bündel an Aufgaben durch eine kosmetische und/oder dermatologische Zubereitung entsprechend den Hauptansprüchen. In den Unteransprüchen sind bevorzugte Ausführungsformen der Zubereitung offenbart. Die Erfindung umfasst darüber hinaus auch die Verwendung der Zubereitungen auf der Haut und/oder dem Haar.

Es hat sich überraschend gezeigt, und darin liegt die Lösung dieser Aufgaben, dass eine kosmetische Zubereitung enthaltend
a.) Proteinhydrolysate aus Seide, Pashmina, Cashmere-Wolle, Merinowolle und/oder Mohair,
b.) Extrakt aus den Muschelfäden der Miesmuscheln, den so genannten Byssus-Fäden,und und
c.) Sericin und/oder Sericinhydrolysate sowie gegebenenfalls
d.) zusätzlich biogene bzw. funktionelle Aminosäuren, wie Carnithin, Kreatin, Taurin und/oder Glutathion.
die Haut- und Haareigenschaften spürbar und sichtbar verbessert.

Dabei ist das Zusammenspiel und die daraus resultierende synergistische Wirkung der speziellen Proteinhydrolysaten aus biogenen Fasern, wie z.B. Pashmina oder Merinowolle, Seide und Muschelbyssos mit osmolytischen Aminosäuren, z.B. Taurin, zur Haar-, Haut- und insbesondere Kopfhautpflege herauszustellen.
Insbesondere die Kombinationen von a.) Casmerewollezubereitungen, b.) Byssosseidenzubereitungen und c.) Seidenbastsericin sowie ggf. den oben bezeichneten biogenen Aminosäuren d.) erwirken eine deutlich wahrnehmbare Verbesserung der Haarstruktur und Sensorik.

Die erfindungsgemäße Kombination der Bestandteile a. - c. sowie ggf. d. zeigt einen Synergismus, der für die überraschenderweise äußerst positiven Eigenschaften der kosmetischen Zubereitung verantwortlich ist. Die Einzelbestandteile sind in einer kosmetischen Zubereitung zu einem Wirkstoffkomplex verbunden und entfalten erst in der erfindungsgemäßen Zusammensetzung ihre vorteilhaften Positiveigenschaften auf das Haar und die Kopfhaut.
In internen Studien konnte gezeigt werden, dass die erfindungsgemäßen Zubereitungen einen taktilen Vorteil, insbesondere in Bezug auf Haarfülle, Geschmeidigkeit und Glanz, bewirken. Diese Vorteile werden durch kosmetische Zubereitungen, die jeweils ein oder zwei der erfindungsgemäßen Einzelkomponenten enthalten, nicht beobachtet.

Die erfindungsgemäßen Kombinationen regen den Stoffwechsel in der Kopfhaut an und fördert die Feuchtigkeitsanreicherung auf der Kopfhaut und im Haar und zeichnen sich vor allem durch gute Haut- und Haarverträglichkeit aus. Ferner liefern die Aminosäurebeslandteile die Faktoren an Haut und Haar zurück, welche wie "Magnete" für die ideale Feuchtigkeit in der Haut und seinen Anhangsgebilden führen. Das wirkt sich insbesondere positiv auf die Frisierbarkeit des Haares aus, welches durch zu wenig oder zu schnell flüchtige Feuchtigkeit seine Frisierbarkeit durch elektrostatische Aufladung zu verlieren droht.
Durch die topische Anwendung der Zubereitung auf der Haut und/oder dem Haar werden insbesondere folgende positive Eigenschaften des behandelten Haares bzw. Haut festgestellt:
➢ Verbesserung der Frisierbarkeit des Haares
➢ Vermeidung des negativ Effektes der statischen Aufladung der Haare
➢ stabilisierenden Effekt auf die Haarstruktur
➢ pflegend für Haar und Kopfhaut, insbesondere über einen längeren Zeitraum nach der Anwendung
➢ strukturglättende Wirkung
➢ Verleiht dem haar natürlichen Schimmer und attraktives Volumen
➢ das Haar ist leichter zu kämmen und einfacher zu stylen
➢ schützt das Haar und die Kopfhaut beim Fönen
➢ schützt vor elektrostatischer Aufladung durch Balancierung und Ausgleich des Feuchtigkeitshaushaltes der Haare

Bevorzugt werden die erfindungsgemäßem Zubereitungen silikonfrei hergestellt und vertrieben.

Auch als Shampoo zeigten sich überraschend positive Eigenschaften, so wirkt das mit den erfindungsgemäßen Zubereitungen behandelte Haar äußerst gepflegt und lässt sich gut frisieren. Dies ist beispielsweise an der Kämmbarkeit, des Anscheins und der Fülle des Haares erkennbar.

Der erfindungsgemäße Bestandteil a.) wird gewählt aus Proteinzubereitungen aus Seide, Pashmina, Cashmere-Wolle, Merinowolle und/oder Mohair.
Pashm ist das persiche Wort für Wolle und Pashmina und beschreibt die feine, flauschige Pashminawolle, die aus den Höhen des tibetanischen Himalayagebirges stammt und gewonnen wird. Als Kosmetische Produkt ist beispielsweise Pashmisilk® bekannt, enthaltend ein Hydrolysat aus Pashmina, Merinowolle und Naturseide.
Proteinhydrolisate der Cashmere-Wolle sind bereits beschrieben. Sie werden beispielsweise von Laboratoires Serobiologiques, Farnkreich, hergestellt und vertrieben und als "Cashmilan" vertrieben..
Proteinhydrolysate der Merinowolle oder Mohair-Wolle sind ebenfalls als erfindungswesentliche Bestandteile vorgesehen. Mohair-Wolle wird von der Angoraziege gewonnen.
Das Proteinhydrolysate aus Pashmina, Cashmere-Wolle, Merinowolle und/oder Mohair umfassen im wesentlichen Aminosäure, Peptid und Keratinhydrolysate
Bevorzugt wird hydrolysiertes Seidenprotein, beispielsweise als Silkpro® der Fa. Ikeda Corp.(CAS Nr.: 96690-41-4) verwendet.

Weniger bekannt ist, dass einige Muschelarten Rohstoff für Kosmetika liefern. Beispielsweise die Muschelseide oder Byssusseide, auch goldene Meerwolle oder Meerseide genannt, in der Vergangenheit Rohstoff für die Mode, den legendären "goldenen Gewänder", gewesen. Allerdings gilt das Wort "byssos" für verschiedene feine Gewebe, so dass erst aus zusätzlichen Bezeichnungen oder den Umständen auf Muschelseide geschlossen werden kann.
Bekannt ist z.B. Mythuline®, ein Extrakt der Muschelfäden der Miesmuschel, hergestellt von Laboratoires Seporga. Der Extrakt enthält Mikrofasern mit alternierenden Gruppen aus Collagen und Elastin die durch Fibroin zusammengehalten werden, wie das folgende Schaubild erläutert.

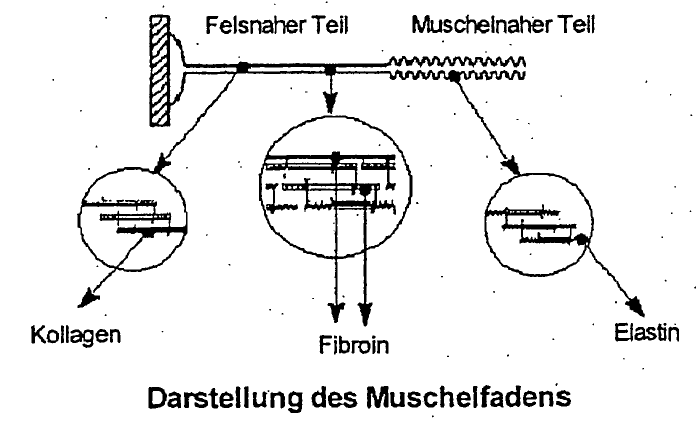

Die bedeutendsten Unterarten der Miesmuscheln sind die Nordseemiesmuschel, die Mittelmeermiesmuschel, die Meerdattel und die Neuseeländische Miesmuschel. All diese Arten sind vorwiegend im Flachwasser verbreitet, wo sie sich mit Hilfe ihres Byssus an Felsen oder aneinander festklammern. Der Name Miesmuschel entstand aus eben diesem Byssus, auch dem sogenannten Moos, den sie als Fangarme benutzen und der durch Lautverschiebung statt Moos- Miesmuschel ergibt.

Viele kleine Bewegungen verursachen im Laufe unseres Lebens eine große mechanische Beanspruchung unserer Haut und Haar. Die Wiederholung dieser Belastungen zusammen mit der Desorganisation der Bindefasern (Glycolisierung, Elastinabbau, Kollagenpolymerisation) führt zu einem Elastizitäts- und Straffheitsverlust der Haut, beschleunigt den Alterungsprozess und damit die Faltenbildung. Muscheln haben ganz ähnliche physikalische Stresssituationen wie unsere Haut bzw. unser Haar zu bewältigen. Ihre Fäden müssen für einen starken Halt am Untergrund sorgen, um den wechselnden Zuständen ihrer Umgebung (Wellen, Trockenfallen, Kampf um den Platz mit anderen Muscheln, Algen) widerstehen zu können. Da die Natur Probleme vorbildlich lösen kann, besteht der Muschelfaden des Mytilus edulis, der Miesmuschel, aus drei starken Proteinmikrofasern (Elastin, Exokollagen, Fibroin), deren spezifische Struktur und biomechanischen Eigenschaften der Faser gleichzeitig Widerstand und Elastizität verleihen.

Dabei wechseln sich Elastin- und Exokollagenpeptid im Faden ab. Das Filament ist vollständig von Fibroin (Seide aus dem Meer) bedeckt. Durch die Lichtbrechung auf ihrer Oberfläche erscheinen die Seidefasern zudem glänzend. Fibroin ist der wesentliche Bestandteil der Seide, eine fadenförmige, flexible und glänzende Substanz. Ähnlich einem Prisma verleiht es der Seide den eigentümlichen, seidigen Effekt. Die Hauptwirkung der Seidenfäden ist die Wiederstandskraft und Elastizität der Miesmuschelfäden zu gewährleisten.
Elastin kann bis zu 160% seiner Länge gedehnt werden ohne zu reißen Elastin ist für die Elastizität der Miesmuschelfäden mitverantwortlich.
Ein Extrakt dieser Fäden, als Muschelfäden der Miesmuscheln, den Byssus-Fäden bezeichnet, führt in Kombination mit den erfindungsgemäßen Bestandteilen a.) und c.) zu einer feuchtigkeitsspendenden und stärkenden Wirkung des Haares. Zudem wird die Kollagen I und III Synthese gesteigert.
Durch den zusätzlichen Effekt des Seideschimmers wird der Glanz des Haares verbessert.

Als dritter wesentlicher Bestandteil der erfindungsgemäßen Zubereitungen hat sich Sericin erwiesen. Sericin ist eine Eiweißsubstanz aus dem Kokon des Seidenspinners, die den Rohseidenfaden umgibt. Bekannt ist beispielsweise Setakol®, ein Hydrolysat des Seidenproteins Sericin, das von der Firma Pentapharm, Basel, hergestellt und vertrieben wird. Es handelt sich dabei um eine wässrige Lösung des Seidenproteins Sericin. Die vorteilhafte Eigenschaft des Sericin, die erfindungsgemäß in Kombination mit den Eigenschaften der Bestanteile a. und b. genutzt werden, ist, dass Sericin sich mit Kreatin der Haut und des Haares verbindet und somit eine schützende Wirkung ermöglicht. Sericin führt damit eine Schutzfunktion für das mit der erfindungsgemäßen Zubereitung behandelten Haare oder Haut aus.

Bevorzugt enthält die erfindungsgemäße Zubereitungen Pashmisilk®, Silkpro® und/oder "Cashmilan" als erfindungsgemäßen Bestandteil a., Mythuline® als Bestandteil b. und Setakol® als Bestandteil c. Diese drei Proteinzubereitungen stellen jeweils erfindungsgemäß bevorzugte Hydrolysate der Einzelbestandteile a., b. und c. dar. Die Kombination führt zu einer deutlich verbesserten Weichheit und Geschmeidigkeit des Haares. Ferner wird der Glanz des Haares deutlich verbessert und durch die Verankerung von Feuchtigkeitsfaktoren im Haar die Frisierbarkeit deutlich verbessert.

Die Zubereitung enthält bevorzugt einen Anteil von 0,001 bis 50 Gew.%, bezogen auf die Gesamtmasse der Zubereitung, insbesondere 0,01 bis 15%, vorzugsweise 0,05 bis 5%, an funktionellen oder biogenen Aminosäuren, vorzugsweise mit den Aminosäuren Taurin und/oder Kreatin.
Die Gewichtsanteile der Komponenten a. - d. untereinander kann bevorzugt folgende Bereiche annehmen.
a. 0,025 - 99 Gew.%, bevorzugt 0,5 - 50 Gew.%
b. 0,025 - 99 Gew.%, bevorzugt 0,5 - 50 Gew.%
c. 0,025 - 99 Gew.%, bevorzugt 0,5 - 50 Gew.%
d. 0 - 50 Gew.%, bevorzugt 0,01 - 15 Gew.%.

Aufgrund der aus den verschiedenen Proteinzubereitungen erhältlichen Bestandteile hat sich als besonders bevorzugt herausgestellt, dass die kosmetische Zubereitungen eine Kombination aus HYDROLYZED COLLAGEN (Bestandteil b.), CAS Nr 92113-31-0), HYDROLYZED ELASTIN (Bestandteil b.), CAS Nr 100085-10-7), HYDROLYZED KERATIN (Bestandteil d.), CAS Nr 69430-36-0), HYDROLYZED SERICIN (Bestandteil c.), CAS Nr 96690-41-4) und HYDROLYZED SILK (Bestandteil a.),CAS Nr 73049-73-7) enthält.

Besonders bevorzugt ist eine kosmetische Zubereitung enthaltend 0,005 - 0,03 Gew.% HYDROLYZED COLLAGEN, 0,005 - 0,03 Gew.% HYDROLYZED ELASTIN, 0,05 - 0,5 Gew.% HYDROLYZED KERATIN, 0,01 - 0,5 Gew.% HYDROLYZED SERICIN und 0,01 - 0,5 Gew.% HYDROLYZED SILK.

Bevorzugt enthält die erfindungsgemäße Zubereitung zusätzlich Taurin. Durch den Zusatz an Taurin wird dessen Osmolytwirkung genutzt, die eine Anreicherung der Feuchtigkeit auf der Kopfhaut und des Haares bewirkt. Dieser Zusatzeffekt führt zu einer weiteren Verbesserung der Frisierbarkeit des Haares.
Durch den Zusatz an Taurin in der erfindungsgemäßen Zubereitung wird eine Zunahme an Feuchtigkeit der Kopfhaut und/oder des Haares festgestellt.
Vor allem lässt sich durch den Zusatz an Taurin der lästige Effekt der statischen Aufladung des Haares vermeiden.

Dabei ist es Gegenstand dieser Erfindung, das überraschenderweise insbesondere Produkte bzw. Zubereitungen aus Produkten von biogenen Aminosäuren, wie z.B. dem Taurin zusammen mit Zubereitungen aus Meeresseide, Pashmina und Seidenbastsericin besonders vorteilhaft anzusehen sind.

Taurin ist eine bedingt essentielle Aminosäure, die in den Geweben der meisten Tierarten zu finden ist. Sie dient nicht als Baustein von Proteinen, sondern ist in vielen Geweben in freier Form vorhanden. Taurin ist an.einer Reihe von physiologischen Prozessen beteiligt, z.B. der Konjugation von Gallensäuren, der Osmoregulation, der Detoxifikation von Xenobiotika, der Stabilisierung von Zellmembranen, der Steuerung des zellulären Kalziumstroms und der Modulation der neuronalen Erregbarkeit. Taurin (2-Aminoethansulfonsäure) unterscheidet sich von anderen Aminosäuren dadurch, dass die Carboxylgruppe durch eine Sulfonsäuregruppe ersetzt ist und dass es nicht in Proteine eingebaut wird. Taurin ist somit keine Aminosäure im engeren Sinn.

Die Herstellung bzw. Bereitstellung der Bestandteile a. - d. ist dem Fachmann bekannt. Die erfindungsgemäßen kosmetischen und dermatologischen Zubereitungen können kosmetische Hilfsstoffe enthalten, wie sie üblicherweise in solchen Zubereitungen verwendet werden.

Beispielsweise kann das Cashmerehydrolysat durch enzymatische Hydrolyse der Cashmerewolle, anschließender Filtration und Konservierung gewonnen werden. Der Extrakt aus den Muschelfäden, den so genannten Byssus-Fäden, wird bevorzugt aus der Muschel Mytilus edulis gewonnen. Die Muschelfäden werden mehrere Stunden bei mindestens 70°C in einer sauren wässrigen Lösung hydrolysiert. Der pH-Wert wird auf 5 - 6 eingestellt und das flüssige Produkt zur Entfärbung mit Aktivkohle gefiltert und anschließend zentrifugiert. Das Produkt wird anschließend mittels Sterilfiltration sterilisiert und 12 h bei 65 °C gelagert.

Ein zusätzlicher Gehalt an Antioxidantien ist in kosmetischen und/oder dermatologischen Zubereitungen im allgemeinen bevorzugt.

Die Mittel gemäß der Erfindung können beispielsweise als aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels.

Als Treibmittel für aus Aerosolbehältern versprühbare kosmetische oder dermatologische Zubereitungen im Sinne der vorliegenden Erfindung, sind die üblichen bekannten leichtflüchtigen, verflüssigten Treibmittel, beispielsweise Diethylether, Kohlenwasserstoffe (Propan, Butan, Isobutan) geeignet, die allein oder in Mischung miteinander eingesetzt werden können. Auch Druckluft, Stickstoff, Stickstoffdioxid oder Kohlendioxid oder Gemische aus diesen Substanzen sind vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Als Haarpflegemittel wird eine Vielzahl von Produkten bezeichnet, deren wichtigste Vertreter Vorbehandlungsmittel, Haarwässer und Haarkurmittel sind.

Grundstoffe für Haarpflegemittel sind z.B. Fettalkohole, Wachse, Paraffine, Vaseline, Paraffinöl und Lösemittel.

Fettalkohole sind z.B. gerad- oder verzweigtkettige aliphatische einwertige Alkohole mit 6 - 22 C-Atomen im Molekül. In der Kosmetik werden vorzugsweise gradkettige Fettalkohole mit einer Kettenlänge von 12 - 18 C-Atomen verwendet. Diese Fettalkohole sind weiche, farblose. Massen, praktisch ungiftig und gut hautverträglich. Fettalkohole werden bevorzugt zur Herstellung von Haarkuren und Frisiercremes verwendet, wobei dem Cetylalkohol und dem Stearylalkohol besondere Bedeutung zukommt.

Wachse sind Fettsäureester, die in tierischen und pflanzlichen Produkten vorkommen, aber auch synthetisch hergestellt werden können. Das wohl bekannteste natürlich vorkommende Wachs ist das Bienenwachs, das als Hauptbestandteile Cerin und Myricin enthält. Wachs ist jedoch ein Oberbegriff für eine Reihe natürlich oder künstlich gewonnener Stoffe, die in der Regel halbfeste, weiße, geruchlose und in Wasser unlösliche Massen darstellen.

Paraffine im kosmetischen Sinn sind weiße, geruchlose Massen aus geradkettigen hochmolekularen Kohlenwasserstoffen. Wegen ihrer den der Wachse vergleichbaren Eigenschaften werden sie auch oft als Erdölwachse bezeichnet.

Vaseline ist ein Gemisch von verzweigtkettigen Paraffinen mit geringem Anteil an zyklischen Paraffinen. Es ist eine weiche, transparente und in Wasser unlösliche Masse mit geringem Eigengeruch, die bei der Aufbereitung des Erdöls anfällt.

Paraffinöl ist ein Gemisch gesättigter flüssiger Kohlenwasserstoffe. Es ist unlöslich in Wasser, aber mischbar mit Fettalkoholen und Wachsen. Es wird als Zusatz in Haarpflegemitteln zur Konsistenzregulierung verwendet.

Lösemittel spielen in der Kosmetik eine erhebliche Rolle. Von der großen Anzahl Lösemittel, die zur Verfügung stehen, hat Ethanol die größte Bedeutung. Es wird zur Herstellung von Haarwässern verwendet, in denen es wegen seiner desinfizierenden Eigenschaften gleichzeitig die Funktion eines Wirkstoffes erfüllt.

Hilfsstoffe können verwendet werden, um bestimmte Eigenschaften der Haarpflegemittel, z.B. Konsistenz, Temperatur- und Lichtstabilität, Aussehen und Geruch, zu verbessern sowie deren Herstellung zu erleichtern. Zugesetzt werden z.B. nach Bedarf:
- Emulgatoren, um die Grenzflächenspannung zwischen zwei an sich nicht mischbaren Phasen so weit herabzusetzen, dass deren feine Vermischung möglich wird,
- Verdickungsmittel, um die Stabilität von Emulsionen zu erhöhen' und deren Viskosität einzustellen,
- UV-Absorber, um die Lichtstabilität der in Haarpflegemitleln enthaltenen Farbstoffe und anderer lichtempfindlicher Komponenten zu verbessern. Daneben dienen sie dem Schutz des Haares vor Lichteinflüssen.
- Konservierungsmittel, um mikrobieller Zersetzung vorzubeugen.
- Antioxidantien, um Geruchsveränderungen, die durch Oxidationsvorgänge hervorgerufen werden können, zu verhindern.
- Farbstoffe, um Haarpflegemitteln ein ansprechendes Aussehen zu verleihen.
- Parfümöle, um Haarpflegemitteln einen angenehmen Duft zu verleihen und Nebengerüche der Rohstoffe zu überdecken.

Quartäre Ammonium-Verbindungen sind eine wichtige Gruppe der speziellen Wirkstoffe, die zur Herstellung von Haarpflegemitteln verwendet werden. Haarpflegemittel, insbesondere Haarkuren, erhalten wesentliche Eigenschaften wie Verbesserung von Kämmbarkeit sowie Griff und Verhinderung statischer Aufladung der Haare vornehmlich durch den Einsatz quartärer Ammonium-Verbindungen. Diese Eigenschaft kann zusätzlich durch den Gehalt an Taurin verbessert werden.
Die Eigenschaften quartärer Ammonium-Verbindungen werden durch die kationische Gruppe einerseits und durch die Art der lipophilen Reste dieser Gruppe andererseits bestimmt. Geeignet sind z.B. die Verbindungen, in denen ein bis zwei Reste längerkettige Alkyl-Gruppen, wie Lauryl-, Cetyl- oder Stearyl-Gruppen, und die verbliebenen Reste Methyl-Gruppen sind. Produkte dieses Typs werden vorzugsweise als Chloride, Bromide und Methosulfate eingesetzt.

Geeignet sind auch polymere quartäre Ammonium-Verbindungen, Makromoleküle, deren wesentliches Merkmal das Vorhandensein mehrerer quartärer Ammonium-Gruppen im Molekül ist. Dadurch wird ihre Haftfähigkeit am Haar deutlich erhöht.

Besonders vorteilhaft zu verwendende kationische Tenside sind
1. Alkylamine
2. Alkylimidazole
3. Ethoxylierte Amine und
4. Quaternäre Tenside
5. Esterquats

Quaternäre Tenside erhalten mindestens ein N-Atom, das mit 4 Alkyl- oder Arylgruppen kovalent verbunden ist. Dies führt, unabhängig vom pH-Wert, zu einer positiven Ladung. Die erfindungsgemäß verwendeten kationischen Tenside können ferner bevorzugt gewählt werden aus der Gruppe der quaternären Ammoniumverbindungen, insbesondere Benzyltrialkylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride der -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.
Monomere oder polymere quartäre Ammonium-Verbindungen werden vielfach in Haarspülungen und Haarkuren z.B. in Konzentrationen von 0,5 - 5 Gew.-% eingesetzt. Dazu gehören Cetrimonium Chloride wie es unter der Bezeichnung Dehyquart A von der Fa. Henkel angeboten wird oder Distearoylethyl Hydroxyethylmonium Methosulfate wie es unter der Bezeichnung Dehyquart F 75 von der Fa. Henkel angeboten wird.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um Emulsionen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine Emulsion dar und enthält die erfindungsgemäßen Kombinationen. Es ist allerdings gegebenenfalls vorteilhaft, wenn die erfindungsgemäße Lotion in Form einer Mikroemulsion oder einer wäßrigen oder wäßrig-alkoholischen Lösung vorliegt.

Erfindungsgemäß können kosmetische Zubereitungen zur Behandlung und Pflege der Haare als Gele vorliegen, die organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die vorstehenden Prozentangaben beziehen sich auf das Gesamtgewicht der Zubereitungen.

Die erfindungsgemäßen Zubereitungen können kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Die Menge der Verdickungsmittel beträgt beispielsweise 0,05 bis 5,0 Gew.-%, vorzugsweise 0,1 bis 3,0 Gew.-%, insbesondere 0,15 bis 2,0 Gew.-%, jeweils bezogen auf das Gesamtgewicht der Zubereitung.

Der Wassergehalt der Zubereitungen beträgt beispielsweise 60 bis 95 Gew.-%, vorzugsweise 75 bis 95 Gew.-%, insbesondere 80 bis 90 Gew.-%, jeweils bezogen auf das Gesamtgewicht der Zubereitung.

Zum Schutz der Formulierung und / oder des zu behandelnden Haares können alle für kosmetische und/oder dermatologische Anwendungen geeigneten oder gebräuchlichen Antioxidantien verwendet werden.

Die Gesamtmenge der Antioxidantien beträgt beispielsweise 0,001 bis 2 Gew.-%, vorzugsweise 0,01 bis 1 Gew.-%, jeweils bezogen auf das Gesamtgewicht der Zubereitung.

Vorteilhaft werden weitere Antioxidanzien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Vorteilhaft können erfindungsgemäße Zubereitungen außerdem Substanzen enthalten, die UV-Strahlung im UV-B-Bereich absorbieren, wobei die Gesamtmenge der Filtersubstanzen z.B. 0,001 Gew.-% bis 30 Gew.-%, vorzugsweise 0,05 bis 10 Gew.-%, insbesondere 0,1 bis 1,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut, insbesondere die Kopfhaut dienen.

Enthalten die erfindungsgemäßen Emulsionen UV-B-Filtersubstanzen, können diese vorteilhaft wasserlöslich sein. Vorteilhafte wasserlösliche UV-B-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxyben-zophenon-5-sulfonsäure und ihre Salze;
- Sulfonsäure-Derivate des 3-Benzylidencamphers, wie z.B. 4-(2-Oxo-3-bornyliden-methyl)benzolsulfonsäure, 2-Methyl-5-(2-oxo-3-bornylidenmethyl)sulfonsäure und ihre Salze.

Es kann auch von Vorteil sein, erfindungsgemäße Zubereitungen mit UV-A-Filtern zu versetzen, die bisher üblicherweise in kosmetischen Zubereitungen enthalten sind. Es können die für die UV-B-Kombination verwendeten Mengen eingesetzt werden.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Der pH-Wert der Zubereitungen kann in bekannter Weise durch Zugabe von Säuren oder Basen eingestellt werden, vorzugsweise durch Zugabe von Puffergemischen, z.B. auf Basis von Citronensäure/Citrat oder Phosphorsäure Phosphat-Puffergemischen. Vorzugsweise liegt der pH-Wert unter 10, z.B. im Bereich von 2-7, insbesondere im Bereich von 3-5.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen. Proteinhydrolysate aus Seide, Pashmina, Cashmere-Wolle, Merinowolle und/oder Mohair, Extrakt aus den Muschelfäden der Miesmuscheln, den so genannten Byssus-Fäden, und Sericin und/oder Sericinhydrolysate sowie gegebenenfalls

### Beispiele

### Beispiel 1 Cleansing-Gel

| | Gew.% |
|---|---|
| Proteinhydrolysat (Seide, Pashmina, Byssus-Fäden, Sericin) | 5,00 |
| Natrium Laureth Sulfat | 13,00 |
| Natrium Cocoamphoacetat | 2,00 |
| Zitronensäure | 0.50 |
| Dicaprylylether | 8,00 |
| Glyceryl Linoleat | 2,50 |
| Glycerin | 5,00 |
| PEG-150 Distearat . | 0,82 |
| Kochsalz | 1.62 |
| Antioxidantien | q.s. |
| Konservierungsmittel | q.s. |
| Wasser | ad 100,000 |

### Beispiel 2 Badezubereitung

| | Gew.% |
|---|---|
| Proteinhydrolysat (Cashmere, Byssus-Fäden, Sericin) | 7,50 |
| Natrium Laureth Sulfat | 13,00 |
| Cocamidopropyl Betain | 2,00 |
| Zitronensäure | 0.50 |
| Dicaprylylether | 8,00 |
| Glyceryl Linoleat | 2,50 |
| Glycerin | 5,00 |
| PEG-150 Distearat | 0,82 |
| Kochsalz | 1.62 |
| Antioxidantien | q.s. |
| Konservierungsmittel | q.s. |
| Wasser | ad 100,000 |

### Beispiel 3 Duschgel

| | Gew.% |
|---|---|
| Proteinhydrolysat (Seide, Pashmina, Byssus-Fäden, Sericin) | 15,00 |
| Natrium Laureth Sulfat | 13,00 |
| Natrium Cocoamphopropionat | 2,00 |
| Zitronensäure | 0,23 |
| Dicaprylylether | 8,00 |
| Glyceryl Linoleat | 3,00 |
| Glycerin | 5,00 |
| PEG-150 Distearat | 0,85 |
| Kochsalz | 1,63 |
| Antioxidantien . | q.s. |
| Konservierungsmittel | q.s. |
| Wasser | ad 100,000 |

### Beispiel 4 Cleansing-Zubereitung

| | Gew.% |
|---|---|
| Proteinhydrolysat (Seide, Merinowolle, Pashmina, Byssus-Fäden, Sericin) | 2,75 |
| Natrium Laureth Sulfat | 9,00 |
| Natrium Cocoamphopropionat | 6,00 |
| Zitronensäure | 0,40 |
| Dicaprylylether | 8,00 |
| Glyceryl Linoleat | 2,50 |
| Glycerin | 5,00 |
| PEG-150 Distearat | 0,83 |
| Kochsalz | 0,81 |
| Antioxidantien | q.s. |
| Konservierungsmittel | q.s. |
| Wasser | ad 100,000 |

### Beispiel 5 Cleansing-Gel

| | Gew.% |
|---|---|
| Proteinhydrolysat . (Seide, Merinowolle, Pashmina, Byssus-Fäden, Sericin) | 12,75 |
| Natrium Laureth Sulfat | 13,00 |
| Dinatrium Cocoyl Glutamat | 2,00 |
| Zitronensäure | 0,65 |
| Coco Caprylat/Caprat | 8,00 |
| Glyceryl Linoleat | 2,50 |
| Glycerin | 5,00 |
| PEG-150 Distearat | 1,00 |
| Kochsalz | 1,63 |
| Antioxidantien | q.s. |
| Konservierungsmittel | q.s. |

### Beispiel 6 Shampoo

| | Gew.% |
|---|---|
| Proteinhydrolysat (Pashmina, Byssus-Fäden, Sericin) | 8,00 |
| Natrium Laureth Sulfat | 9,00 |
| Natrium Cocoamphoacetat | 6,00 |
| Zitronensäure | 1,20 |
| Paraffinum liquidum | 2,00 |
| Glyceryl Linoleat | 1,00 |
| Glycerin | 5,00 |
| PEG-150 Distearat | 0,30 |
| Antioxidantien | q.s. |
| Konservierungsmittel | q.s. |
| Wasser | ad 100,000 |

### Beispiel 7 Schäumendes Duschgel

| | Gew.% |
|---|---|
| Proteinhydrolysat (Pashmina, Byssus-Fäden, Sericin) | 3,00 |
| Natrium Laureth Sulfat . | 9,00 |
| Natrium Cocoamphoacetat | 6,00 |
| Zitronensäure | 1,20 |
| Paraffinum liquidum | 2,00 |
| Glyceryl Isostearat | 1,00 |
| Glycerin | 5,00 |
| PEG-150 Distearat | 0,30 |

### Beispiel 8 Shampoo

| | Gew.% |
|---|---|
| Proteinhydrolysat (Pashmina, Byssus-Fäden, Sericin, Taurin, Kreatin) | 7,00. |
| Natrium Laureth Sulfat | 9,00 |
| Natrium Cocoamphoacetat | 6,00 |
| Zitronensäure | 1,20 |
| Paraffinum liquidum | 2,0 |
| Sorbitanisostearat | 1,00 |
| Glycerin | 5,00 |
| PEG-150 Distearat | 0,30 |

### Beispiele 9, 10, 11, Conditioner-Shampoo mit Perlglanz

| | 9 | 10 | 11 |
|---|---|---|---|
| Proteinhydrolysat | | | |
| (Cashmere, Seide, Byssus-Fäden, Sericin, Taurin) | 5,5 | 4,5 | 9,0 |
| Polymer JR 400¹ | 0,5 | 0,5 | 0,5 |
| Texapon K 14 S spezial² | 4,0 | 5,0 | 4,5 |
| Plantacare 2000³ | 9,0 | 8,0 | 8,5 |
| Perlglanzmittel | 2,0 | 2,0 | 2,0 |
| Rewopol SBCS 50⁴ | 6,0 | 5,5 | 6,2 |
| Atlas G-4280⁵ | 3,2 | 3,5 | 3,0 |
| Konservierungsmittel, Parfüm, Verdicker, pH-Einstellung und Lösungsvermittler | q.s. | q.s. | q.s. |
| Wasser, VES (vollentsalzt) | ad 100,0 | ad 100,0 | ad 100,0 |

| | | | |
|---|---|---|---|
| ¹ kationisches Cellulosederivat der Fa. Amerchol, ² Myrethsulfat 70%ig der Fa. Cognis (AES) ³ Decylgucoside 50%ig der Fa. Cognis (APG) ⁴ Citronensäurealkylpolyglykolester-sulfosuccinat, Dinatriumsalz 38%ig der Fa. Goldschmidt (ACS) ⁵ PEG-80 Sorbitan Laurate 73%ig der Fa. Uniqema (PSE) | | | |

### Beispiel 12 - 16 PIT - Emulsionen

| Beispiele | 12 | 13 | 14 | 15 | 16 |
|---|---|---|---|---|---|
| Proteinhydrolysat (Pashmina, Seide, Byssus-Fäden, Sericin) | 15,0 | 8,5 | 2,0 | 17,0 | 12,0 |
| Glycerinmonostearat selbstemulgierend | 0,50 | | 3,00 | 2,00 | 4,00 |
| Polyoxyethylen(12)cetylstearylether | | 5,00 | | 1,00 | 1,50 |
| Polyoxyethylen(20)cetylstearylether | | | | 2,00 | |
| Polyoxyethylen(30)cetylstearylether | 5,00 | | 1,00 | | |
| Stearylalkohol | | | 3,00 | | 0,50 |
| Cetylalkohol | 2,50 | 1,00 | | 1,50 | |
| 2-Ethylhexyl Methoxyzinnamat | | | | 5,00 | 8,00 |
| 2,4-Bis-(4-(2-ethyl-hexyloxy-)2-hydroxyl)-phenyl)-6-(4-methoxyphenyl)-(1,3,5)-triazin | | 1,50 | | 2,00 | 2,50 |
| 1-(4-tert-Butylphenyl)-3-(4-methoxyphenyl)-1,3-proparidion | | | 2,00 | | |
| Diethylhexyl Butamidotriazon | 1,00 | 2,00 | | 2,00 | |
| Ethylhexyl Triazon | 4,00 | | 3,00 | 4,00 | |
| 4-Methylbenzyliden Campher | | 4,00 | | | 2,00 |
| Octocrylen | | 4,00 | | | 2,50 |
| Phenylen-1,4-bis-(mononatrium, benzimidazyl-5,7-disulfonsaeure | | | 0,50 | | 1,50 |
| Phenylbenzimidazol Sulfonsäure | 0,50 | | | 3,00 | |
| C12-15 Alkyl Benzoat | | 2,50 | | | 5,00 |
| Titandioxid | 0,50 | 1,00 | | 3,00 | 2,00 |
| Zinkoxid | 2,00 | | 3,00 | 0,50 | 1,00 |
| Dicaprylylether | | | 3,50 | | |
| Butylenglycol-Dicaprylat/-Dicaprat | 5,00 | | | 6,00 | |
| Dicaprylylcarbonat | | | 6,00 | | 2,00 |
| Dimethicon Polydimethylsiloxan | | 0,50 | 1,00 | | |
| Phenylmethylpolysiloxan | 2,00 | | | 0,50 | 0,50 |
| Shea-Butter (Sheabutter) | | 2,00 | | | 0,50 |
| PVP Hexadecencopolymer | 0,50 | | | 0,50 | 1,00 |
| Glycerin | 3,00 | | 4,00 | 3,00 | 2,00 |
| Tocopherolacetat | 0,50 | | 0,25 | | 1,00 |
| Serinol | 2,00 | 5,00 | 1,00 | 2,00 | 3,00 |
| Milchsäure | 2,40 | 6,00 | 1,20 | | 3,60 |
| Taurin | | 0,3 | | 0,5 | |
| Meersalz | 5,00 | 2,50 | | | |
| Alpha-Glucosylrutin | 0,10 | | 0,20 | | |
| Konservierungsmittel | q.s. | q.s. | q.s. | q.s. | q.s. |
| Ethanol | 3,00 | 2,00 | 1,50 | | 1,00 |
| Parfum | q.s. | q.s. | q.s. | q.s. | q.s. |
| Wasser | ad. 100 | ad. 100 | ad. 100 | ad. 100 | ad. 100 |

### Beispiele 17 - 21 O/W-Creme

| Beispiele | 17 | 18 | 19 | 20 | 21 |
|---|---|---|---|---|---|
| Proteinhydrolysat (Mohair,Cashmere, Seide, Byssus-Fäden, Sericin | 4,00 | 13,5 | 0,8 | 26,00 | 7,00 |
| Glycerylstearatcitrat | | | 2,00 | | 2,00 |
| Glycerylsterat selbstemulgierend | 4,00 | 3,00 | | | |
| PEG-40-Stearat | 1,00 | | | | |
| Polyglyceryl-3-Methylglucose-Distearat | | | | 3,00 | |
| Sorbitanstearat | | | | | 2,00 |
| Stearinsäure | | | | | |
| Polyoxyethylen(20)-cetylstearylether | | | | | |
| Stearylalkohol | | | 5,00 | | |
| Cetylalkohol | 3,00 | 2,00 | | 3,00 | |
| Cetylstearylalkohol | | | | | 2,00 |
| C12-15 Alkylbenzoat | | | | | |
| Caprylic-/Capric-Triglycerid | 5,00 | 3,00 | 4,00 | 3,00 | 3,00 |
| Octyldodecanol | | | 2,00 | | 2,00 |
| Dicaprylylether | | 4,00 | | 2,00 | 1,00 |
| Paraffinum liquidum | 5,00 | 2,00 | | 3,00 | |
| Titandioxid | | | 1,00 | | |
| 4-Methylbenzyliden Campher | | | 1,00 | | |
| 1-(4-tert-Butylphenyl)-3-(4-methoxyphenyl)-1,3-propandion | | | 0,50 | | |
| Serinol | 5,00 | 4,00 | 5,00 | 1,00 | 2,00 |
| Milchsäure | | 4,80 | 6,00 | 1,20 | |
| Meersalz | 5,00 | | | | |
| Taurin | 0,3 | | 0,9 | 0,3 | |
| Betain | | | | | 1,00 |
| Tocopherol | 0,1 | | | | 0,20 |
| Biotin | | | 0,05 | | |
| Ethylendiamintetraessigsaeure Trinatrium | 0,1 | | 0,10 | 0,1 | |
| Konservierungsmittel | q.s. | q.s. | q.s. | q.s. | q.s. |
| Xanthan Gummi | | | | | |
| Polyacrylsaeure | 3,00 | 0,1 | | 0,1 | 0,1 |
| Natronlauge 45% | q.s | q.s. | q.s. | q.s. | q.s. |
| Glycerin | | 1,00 | 1,00 | 5,00 | 3,00 |
| Butylenglycol | | 3,00 | | | |
| Parfum | q.s. | q.s. | q.s. | q.s. | q.s. |
| Wasser | ad 100 | Ad 100 | Ad 100 | Ad 100 | Ad 100 |

### Beispiel 22 Pflegezubereitung

### Proteinhydrolysat

(Pahmina, Cashmere, Seide, Byssus-Fäden, Sericin) in Form einer Mischung aus Pashmisilk®, Silkpro®, Mythuline®, Setakol® sowie Wasser.

### Beispiel 23 Shampoo

| | % w/w |
|---|---|
| BENZOIC ACID | 0,1375 |
| CALCIUM CHLORIDE | 0,0378 |
| CITRIC ACID | 0,5000 |
| DISODIUM PEG-5 LAURYLCITRATE SULFOSUCCINATE | 2,2320 |
| FRAGRANCE (PARFUM) | 0,6000 |
| HYDROLYZED COLLAGEN | 0,0166 |
| HYDROLYZED ELASTIN | 0,0167 |
| HYDROLYZED KERATIN | 0,1500 |
| HYDROLYZED SERICIN | 0,0700 |
| HYDROLYZED SILK | 0,0667 |
| METHYLPARABEN | 0,0038 |
| NIACINAMIDE | 0,1000 |
| ORYZANOL | 0,0500 |
| PANTHENOL | 0,3000 |
| PEG-200 HYDROGENATED GLYCERYL PALMATE | 0,4500 |
| PEG-3 DISTEARATE | 2,0000 |
| PEG-40 HYDROGENATED CASTOR OIL | 0,3000 |
| PEG-7 GLYCERYL COCOATE | 0,0500 |
| PHENOXYETHANOL | 0,0107 |
| POLYQUATERNIUM-10 | 0,5000 |
| SODIUM BENZOATE | 0,2950 |
| SODIUM CHLORIDE | 1,2000 |
| SODIUM COCOAMPHOACETATE | 2,1900 |
| SODIUM LAURETH SULFATE | 10,2180 |
| SODIUM SALICYLATE | 0,4000 |
| TAURINE | 0,1000 |
| TETRASODIUM IMINODISUCCINATE | 0,5000 |
| WATER (AQUA) | 77,5052 |

### Beispiel 24 Rich Maske

| | % w/w |
|---|---|
| CALCIUM CHLORIDE | 0,0378 |
| CETEARYL ALCOHOL | 6,0000 |
| CETRIMONIUM CHLORIDE | 1,0000 |
| FRAGRANCE (PARFUM) | 0,5000 |
| GLYCERIN | 3,8700 |
| HYDROLYZED COLLAGEN | 0,0249 |
| HYDROLYZED ELASTIN | 0,0251 |
| HYDROLYZED KERATIN | 0,2250 |
| HYDROLYZED SERICIN | 0,1050 |
| HYDROLYZED SILK | 0,1001 |
| LANOLIN | 2,0000 |
| LANOLIN ALCOHOL | 0,5000 |
| METHYLPARABEN | 0,2056 |
| NIACINAMIDE | 0,1000 |
| ORYZANOL | 0,0500 |
| PANTHENOL | 0,6000 |
| PHENOXYETHANOL | 0,0161 |
| PHYTANTRIOL | 0,1000 |
| PROPYLPARABEN | 0,1008 |
| SODIUM CITRATE | 0,0350 |
| TAURINE | 0,2000 |
| WATER (AQUA) | 84,2046 |

### Beispiel 25 Styling spray

| | % w/w |
|---|---|
| CALCIUM CHLORIDE | 0,0151 |
| CETEARETH-20 | 0,1500 |
| CETRIMONIUM CHLORIDE | 0,0500 |
| CITRIC ACID | 0,0100 |
| FRAGRANCE (PARFUM) | 0,3000 |
| HYDROLYZED COLLAGEN | 0,0083 |
| HYDROLYZED ELASTIN | 0,0084 |
| HYDROLYZED KERATIN | 0,0750 |
| HYDROLYZED SERICIN | 0,0350 |
| HYDROLYZED SILK | 0.0334 |
| LAURTRIMONIUM CHLORIDE | 0,0075 |
| METHYLPARABEN | 0,0019 |
| NIACINAMIDE | 0,0200 |
| PANTHENOL | 0,3750 |
| PEG/PPG-15/15 DIMETHICONE | 1,0000 |
| PEG-40 HYDROGENATED CASTOR OIL | 0,6000 |
| PHENOXYETHANOL | 0,0054 |
| POLYQUATERNIUM-11 | 0,0600 |
| SODIUM BENZOATE | 0,2000 |
| SODIUM BISULFITE | 0,0150 |
| TAURINE | 0,0500 |
| VP/VA COPOLYMER | 3,7500 |
| WATER (AQUA) | 93,2300 |

### Beispiel 26 Conditioner

| | % w/w |
|---|---|
| ALPHA-ISOMETHYL IONONE | 0,0276 |
| BENZYL SALICYLATE | 0,0131 |
| BIS-DIGLYCERYL POLYACYLADIPATE-2 | 1,6000 |
| BUTYLPHENYL METHYLPROPIONAL | 0,0104 |
| CALCIUM CHLORIDE | 0,0378 |
| CETEARYL ALCOHOL | 2,0000 |
| CETRIMONIUM CHLORIDE | 1,0000 |
| CITRONELLOL | 0,0091 |
| COUMARIN | 0,0075 |
| EUGENOL | 0,0080 |
| FRAGRANCE (PARFUM) | 0,2970 |
| GERANIOL | 0,0113 |
| GLYCERIN | 3,8700 |
| HYDROLYZED COLLAGEN | 0,0083 |
| HYDROLYZED ELASTIN | 0,0084 |
| HYDROLYZED KERATIN | 0,0750 |
| HYDROLYZED SERICIN | 0,0350 |
| HYDROLYZED SILK | 0,0334 |
| HYDROXYISOHEXYL 3-CYCLOHEXENE CARBOXALDEHYDE | 0,0075 |
| HYDROXYPROPYL METHYLCELLULOSE | 0,2500 |
| LINALOOL | 0,0081 |
| METHYLPARABEN | 0,2527 |
| NIACINAMIDE | 0,1000 |
| ORYZANOL | 0,0500 |
| PANTHENOL | 0,3000 |
| PHENOXYETHANOL | 0,0054 |
| PROPYLENE GLYCOL | 0,5000 |
| PROPYLPARABEN | 0,1003 |
| SODIUM CITRATE | 0,0375 |
| TAURINE | 0,1000 |
| WATER (AQUA) | 89,2366 |

## Patentansprüche

1. Kosmetische Zubereitung enthaltend
a.) Proteinhydrolysate aus Seide, Pashmina, Cashmere-Wolle, Merinowolle und/oder Mohair,
b.) Extrakt aus den Muschelfäden der Miesmuscheln, den Byssus-Fäden, und
c.) Sericin und/oder Sericinhydrolysate und gegebenenfalls
d.) biogene bzw. funktionelle Aminosäuren

2. Zubereitung nach Anspruch 1 wobei der Bestandteil d. gewählt wird aus der Gruppe Camithin, Kreatin, Taurin und/oder Glutathion.

3. Zubereitung nach einem der Ansprüche 1 bis 2, **dadurch gekennzeichnet, dass** die Gewichtsanteile der Komponenten a. - d. folgende Bereiche annehmen
a.) 0,025 - 99 Gew.%, bevorzugt 0,5 - 50 Gew.%, insbesondere 1 - 25 Gew.%
b.) 0,025 - 99 Gew.%, bevorzugt 0,5 - 50 Gew.%, insbesondere 1 - 25 Gew.%
c.) 0,025 - 99 Gew.%, bevorzugt 0,5 - 50 Gew.%, insbesondere 1 - 25 Gew.%
d.) 0 - 50 Gew.%, bevorzugt 0,01 - 15 Gew.%, , insbesondere 0,1 - 10 Gew.%, bezogen auf die Gesamtmasse der Zubereitung.

4. Zubereitung nach einem der Ansprüche 1 bis 3, **dadurch gekennzeichnet, dass** die Zubereitung gewählt werden aus der Gruppe wasserhaltiger Zubereitungen, O/W-, W/O-, W/O/W-Emulsionen, Gele, Hydrodispersionen, wässrigen Tensidlösungen, Mikroemulsionen und/oder Mischungen daraus.

5. Mittel nach einem der vorstehenden Ansprüche, **dadurch gekennzeichnet, dass** zusätzlich Antioxidantien mit einem Anteil von 0,001 bis 30 Gew.%, besonders bevorzugt 0,05 - 20 Gew.%, insbesondere 0,1 - 10 Gew.%, bezogen auf das Gesamtgewicht des Mittels, bevorzugt einer kosmetischen Zubereitung, zugesetzt sind.

6. Kosmetische und/oder dermatologische Zubereitung nach einem der vorstehenden Ansprüche, **dadurch gekennzeichnet, dass** zusätzlich Konservierungsmittel, Bakterizide, Parfüme, Substanzen zum Verhindern des Schäumens, Farbstoffe, Füllstoffe, Pigmente, die eine färbende Wirkung haben, Verdickungsmittel, anfeuchtende und/oder feuchthaltende Substanzen, Fette, Öle, Wachse, Alkohole, Polyole, Polymere, Schaumstabilisatoren, Elektrolyte, organische Lösungsmittel, Silikonderivate, Moisturizer, Vitamine, Proteine, Lichtschutzmittel, Stabilisatoren, Insektenrepellentien, Wasser, Salze, antimikrobiell; proteolytisch oder keratolytisch wirksame Substanzen, Medikamente oder andere übliche Bestandteile einer kosmetischen oder dermatologischen Formulierung enthalten sind.

7. Verwendung der Zubereitung nach einem der Ansprüche 1 bis 6 zur topischen Anwendung auf Haut und/oder Haaren.

8. Verwendung der Zubereitung nach einem der Ansprüche 1 bis 6 als Haarpflegezubereitungen, Shampoo oder Haarpflegemittel.

## Claims

1. Cosmetic preparation comprising
a.) protein hydrolyzates from silk, pashmina, cashmere wool, merino wool and/or mohair,
b.) extract from the mussel threads of mussels, the byssus threads, and
c.) sericin and/or sericin hydrolyzates and optionally
d.) biogenic and/or functional amino acids.

2. Preparation according to Claim 1, where the constituent d. is chosen from the group carnithine, creatine, taurine and/or glutathione.

3. Preparation according to one of Claims 1 to 2, **characterized in that** the weight fractions of components a. - d. assume the following ranges
a.) 0.025 - 99% by weight, preferably 0.5 - 50% by weight, in particular 1 - 25% by weight,
b.) 0.025 - 99% by weight, preferably 0.5 - 50% by weight, in particular 1 - 25% by weight,
c.) 0.025 - 99% by weight, preferably 0.5 - 50% by weight, in particular 1 - 25% by weight,
d.) 0 - 50% by weight, preferably 0.01 - 15% by weight, in particular 0.1 - 10% by weight, based on the total mass of the preparation.

4. Preparation according to one of Claims 1 to 3, **characterized in that** the preparation is chosen from the group of hydrous preparations, O/W, W/O, W/O/W emulsions, gels, hydrodispersions, aqueous surfactant solutions, microemulsions and/or mixtures thereof.

5. Composition according to one of the preceding claims, **characterized in that** additionally antioxidants are added in an amount of from 0.001 to 30% by weight, particularly preferably 0.05 - 20% by weight, and in particular 0.1 - 10% by weight, based on the total weight of the composition, preferably a cosmetic preparation.

6. Cosmetic and/or dermatological preparation according to one of the preceding claims, **characterized in that** additionally preservatives, bactericides, perfumes, substances for preventing foaming, dyes, fillers, pigments which have a coloring effect, thickeners, moisturizers and/or humectant substances, fats, oils, waxes, alcohols, polyols, polymers, foam stabilizers, electrolytes, organic solvents, silicone derivatives, moisturizers, vitamins, proteins, photoprotective agents, stabilizers, insect repellants, water, salts, antimicrobially, proteolytically or keratolytically effective substances, medicaments or other customary constituents of a cosmetic or dermatological formulation are present.

7. Use of the preparation according to one of Claims 1 to 6 for tropical application to skin and/or hair.

8. Use of the preparation according to one of Claims 1 to 6 as hair care preparations, shampoo or hair care composition.

## Revendications

1. Préparation cosmétique contenant
a) des hydrolysats de protéines de soie, pashmina, laine-cachemire, laine mérinos et/ou mohair,
b) un extrait des soies marines des moules, les fibres de byssus, et
c) de la séricine et/ou des hydrolysats de séricine et éventuellement
d) des acides aminés fonctionnels ou biogènes.

2. Préparation selon la revendication 1, dans laquelle le composant d est choisi dans le groupe constitué par la carnitine, la créatine, la taurine et/ou le glutathion.

3. Préparation selon la revendication 1 ou 2, **caractérisé en ce que** les proportions en poids des composants a - d se trouvent dans les plages suivantes
a) 0,025-99 % en poids, de préférence 0,5-50 % en poids, en particulier 1-25 % en poids
b) 0,025-99 % en poids, de préférence 0,5-50 % en poids, en particulier 1-25 % en poids
c) 0,025-99 % en poids, de préférence 0,5-50 % en poids, en particulier 1-25 % en poids
d) 0-50 % en poids, de préférence 0,01-15 % en poids, en particulier 0,1-10 % en poids,
par rapport à la masse totale de la préparation.

4. Préparation selon l'une quelconque des revendications 1 à 3, **caractérisée en ce que** la préparation est choisie dans le groupe constitué par les préparations aqueuses, les émulsions H/E, E/H, E/H/E, les gels, les hydrodispersions, les solutions aqueuses de tensioactifs, les microémulsions et/ou des mélanges de telles préparations.

5. Composition selon l'une quelconque des revendications précédentes, **caractérisé en ce qu'**on ajoute en outre des antioxydants en une proportion de 0,001 à 30 % en poids, de façon particulièrement préférée de 0,05-20 % en poids, en particulier de 0,1-10 % en poids, par rapport au poids total de la composition, de préférence d'une préparation cosmétique.

6. Préparation cosmétique et/ou dermatologique selon l'une quelconque des revendications précédentes, **caractérisée en ce que** sont en outre contenus des conservateurs, des bactéricides, des parfums, des substances destinées à empêcher la formation de mousse, des colorants, des charges, des pigments qui ont un effet colorant, des épaississants, des substances humectantes et/ou retenant l'humidité, des graisses, des huiles, des cires, des alcools, des polyols, des polymères, des stabilisants de mousse, des électrolytes, des solvants organiques, des dérivés de silicone, des agents hydratants, des vitamines, des protéines, des photoprotecteurs, des stabilisants, des insectifuges, de l'eau, des sels, des substances à action antimicrobienne, protéolytique ou kératolytique, des médicaments ou d'autres composants usuels d'une composition cosmétique ou dermatologique.

7. Utilisation de la préparation selon l'une quelconque des revendications 1 à 6, pour l'application locale sur la peau et/ou les cheveux.

8. Utilisation de la préparation selon l'une quelconque des revendications 1 à 6, en tant que préparations pour le soin des cheveux, shampooing ou produit de soin capillaire.
